# EUROPEAN PATENT APPLICATION

(11) **EP 4 559 511 A1**
(43) Date of publication of application: **28.05.2025**
(21) Application number: 22951941.8
(22) Date of filing: 20.07.2022
(51) Int. Cl.: A61N 1/36

(54) **ELECTRICAL STIMULATOR**

(71) Applicant: Yamakawa, Kensuke, Kuwana-shi, Mie 511-0947 (JP)
(72) Inventor: Yamakawa, Kensuke, Kuwana-shi, Mie 511-0947 (JP)
(74) Representative: Jones, Nicholas Andrew
(86) International application number: PCT/JP2022/028158
(87) International publication number: WO 2024/018554

(57) **Abstract**

The present invention provides an electrical stimulator capable of more effectively improving brain dysfunction. An electrical stimulator according to the present invention includes: a main body with at least two channels for outputting a set electric signal from a positive electrode output unit and a negative electrode output unit; at least two electrode pairs removably attached to a living body for applying the electric signal to the living body; and a cable for connecting at least two of channels of the main body and at least two electrodes to each other, wherein the two electrode pairs enable electrical stimulation to be applied to the brain while bypassing the brain stem by mounting one electrode pair to two specific positions on the right half of the body and mounting the other electrode pair to two specific positions on the left half of the body, the two electrode pairs are visually distinguishable from each other so that a difference in application of such mounting can be recognized, and the two electrode pairs apply mutually different electric signals to the specific positions.

## Description

### Technical Field

The present invention relates to a technique for enabling recovery from brain dysfunction using an electrical stimulator that applies electric signals to a living body.

### Background Art

Patent Literature 1 describes an electrical stimulator for enabling recovery from brain dysfunction which can stimulate the brain of a living body by intermittently applying electric signals to the living body in a state where electrodes are positioned on at least one part of the palm of a hand, the back of the hand, the sole of a foot, or the top of the foot (for example, the ball of the thumb and a digit of the hand).

Patent Literature 2 describes an electrical stimulator including head electrodes that are mounted to specific positions in a region from the neck to the top of the head of a patient where stimuli can be applied to the brain while bypassing the brain stem (for example, at least one and preferably at least two locations of the temple, the forehead above the eye, the cheek, and the back of the neck) and, preferably, further including an auxiliary electrode that is mounted to a region on a lower body side than the patient's neck (for example, the back). Patent Literature 2 also describes applying a weak current that repeats a predetermined cycle to the head electrodes of the electrical stimulator and adopting a setting where a plurality of the head electrodes, or the head electrodes and the auxiliary electrode, gradually shift from synchronization and synchronize once again at a fixed period.

### Citation List

### Patent Literature

Patent Literature 1
   JP 2009-153904 A (Japanese Patent No. 5053826)
Patent Document 2
   Japanese Patent No. 5345256

### Summary of Invention

### Technical Problem

An object of the present invention is to provide an electrical stimulator capable of more effectively improving brain dysfunction.

### Solution to Problem

The present inventor found that an effect of improving brain dysfunction can be enhanced by configuring an electric signal applied from the electrical stimulator so as to increase an amount of electricity or, in other words, information that passes through the corpus callosum. More specifically, the present inventor found that by using a pair of (a set of two) electrodes to be mounted to two locations of the right half of the body (for example, the right neck and head area) and a pair of electrodes to be mounted to two locations of the left half of the body (for example, the left neck and head area) and using electric signals that differ from each other as an electric signal to be applied from the electrodes for the right half of the neck and head area and an electric signal to be applied from the electrodes for the left half of the neck and head area, amounts of information sent from the left and right hemispheres of the brain to the opposite hemisphere via the corpus callosum can be increased, and such an increase in the amounts of information via the corpus callosum can activate the metabolism of nerve fibers in the corpus callosum, resulting in an enlargement of the nerve fibers and other effects that conceivably improve brain dysfunction.

In other words, the present invention encompasses at least the following invention that relates to an "electrical stimulator".
[Item 1] An electrical stimulator, including:
   a main body with at least two channels for outputting a set electric signal from a positive electrode output unit and a negative electrode output unit;
   at least two electrode pairs removably attached to a living body for applying the electric signal to the living body; and
   a cable for connecting the at least two channels of the main body and the at least two electrode pairs to each other, wherein
   the two electrode pairs enable electrical stimulation to be applied to the brain while bypassing the brain stem by mounting one electrode pair to two specific positions on the right half of the body and mounting the other electrode pair to two specific positions on the left half of the body, the two electrode pairs are visually distinguishable from each other so that a difference in application of such mounting can be recognized, and the two electrode pairs apply mutually different electric signals to the specific positions.
[Item 2] The electrical stimulator according to item **1,** wherein the specific positions include two locations in the neck and head area selected from the group consisting of the temple, the forehead above the eye, the cheek, and the back of the neck.
[Item 3] The electrical stimulator according to item **2,** wherein the two electrode pairs are arranged in advance at positions corresponding to the specific positions including two locations in the neck and head area in a headgear-type fitting to be worn on a wearer's head.
[Item 4] The electrical stimulator according to item 2 or 3, wherein a current value of the electric signal applied to the specific positions including two locations in the neck and head area is set within a range of 1 to 1000 µA.
[Item 5] The electrical stimulator according to any one of items 1 to 4, wherein the specific positions include two locations other than the neck and head area selected from the group consisting of a region from the palm to the back of a hand, a region from the sole to the top of a foot, and a region of the back along the spine.
[Item 6] The electrical stimulator according to item 5, wherein the two electrode pairs are arranged in advance at positions corresponding to the specific positions including two locations other than the neck and head area in a semi-glove-like or semi-sock-like fitting to be worn on a wearer's hand or foot.
[Item 7] The electrical stimulator according to item 5 or 6, wherein a current value of the electric signal applied to the specific positions including two locations other than the neck and head area is set within a range of 1 to 1000 µA.
[Item 8] The electrical stimulator according to any one of items 1 to 7, wherein
   the electric signal applied to the specific positions is:
   (1) set so as to repeat a cycle including a first state in which positively-applied and negatively-applied pulse waves are alternately generated and a second state in which the pulse waves are inactive; or
   (2) set so as to include, in this order, a first state in which a pulse wave that is always positively or negatively applied is generated, a second state in which the pulse wave is inactive, a third state in which a pulse wave applied in reverse to the first state is generated, and a fourth state in which the pulse wave is inactive.
[Item 9] The electrical stimulator according to item 8, wherein
   an electric signal applied from one electrode pair of the two electrode pairs and an electric signal applied from the other electrode pair are
   set such that a duration of either one or both of the first state and the second state in the electric signal described in (1) differs between the two electrodes, or
   set such that a duration of at least one of the first state, the second state, the third state, and the fourth state in the electric signal described in (2) differs between the two electrodes.
[Item 10] The electrical stimulator according to item 8, wherein
   an electric signal applied from one electrode pair of the two electrode pairs and an electric signal applied from the other electrode pair are
   set such that the application of a pulse wave in the first state is reversed between the two electrodes and the application of a pulse wave in the fourth state is reversed between the two electrodes in the electric signal described in (2).
[Item 11] The electrical stimulator according to any one of items 1 to 10, wherein a frequency of the electric signal is set within a range of 10 to 80 pps (pulses per second).
[Item 12] The electrical stimulator according to any one of items 1 to 11, wherein the electrical stimulator is a medical device for treating brain dysfunction.

In addition, the invention that relates to the "electrical stimulator" described above can be converted into an invention that relates to an "electrical stimulation method". In other words, the present invention also encompasses the following invention that relates to an "electrical stimulation method".
[Item 1'] An electrical stimulation method that uses at least two electrode pairs, wherein
   the two electrode pairs enable electrical stimulation
   to be applied to the brain while bypassing the brain stem by mounting one electrode pair to two specific positions on the right half of the body and mounting the other electrode pair to two specific positions on the left half of the body, and
   the two electrode pairs apply mutually different electric signals to the specific positions.
[Item 1A'] The electrical stimulation method according to item 1', wherein
   the two electrode pairs are included in an electrical stimulator that includes:
   a main body with at least two channels for outputting a set electric signal from a positive electrode output unit and a negative electrode output unit;
   a cable for connecting the at least two channels of the main body and the at least two electrode pairs to each other; and
   at least two electrode pairs removably attached to a living body for applying the electric signal to the living body.
[Item 1B'] The electrical stimulation method according to item 1' or item 1'A, wherein the two electrode pairs are visually distinguishable from each other so that a difference in application of mounting can be recognized.
[Item 2'] The electrical stimulation method according to item 1', 1'A, or 1'B, wherein the specific positions include two locations in the neck and head area selected from the group consisting of the temple, the forehead above the eye, the cheek, and the back of the neck.
[Item 3'] The electrical stimulation method according to item 2'**,** wherein the two electrode pairs are arranged at positions corresponding to the specific positions including two locations in the neck and head area by means of a headgear-type fitting to be worn on a wearer's head.
[Item 4'] The electrical stimulation method according to item 2' or 3', wherein a current value of the electric signal applied to the specific positions including two locations in the neck and head area is set within a range of 1 to 1000 µA.
[Item 5'] The electrical stimulation method according to items 1' to 4'**,** wherein the specific positions include two locations other than the neck and head area selected from the group consisting of a region from the palm to the back of a hand, a region from the sole to the top of a foot, and a region of the back along the spine.
[Item 6'] The electrical stimulation method according to item 5'**,** wherein the two electrode pairs are arranged in advance at positions corresponding to the specific positions including two locations other than the neck and head area in a semi-glove-like or semi-sock-like fitting to be worn on a wearer's hand or foot.
[Item 7'] The electrical stimulation method according to item 5' or 6'**,** wherein a current value of the electric signal applied to the specific positions including two locations other than the neck and head area is set within a range of 1 to 1000 µA.
[Item 8'] The electrical stimulation method according to any one of items 1' to 7', wherein
   the electric signal applied to the specific positions is:
   (1) set so as to repeat a cycle including a first state in which positively-applied and negatively-applied pulse waves are alternately generated and a second state in which the pulse waves are inactive; or
   (2) set so as to include, in this order, a first state in which a pulse wave that is always positively or negatively applied is generated, a second state in which the pulse wave is inactive, a third state in which a pulse wave applied in reverse to the first state is generated, and a fourth state in which the pulse wave is inactive.
[Item 9'] The electrical stimulation method according to item 8', wherein
   an electric signal applied from one electrode pair of the two electrode pairs and an electric signal applied from the other electrode pair are
   set such that a duration of either one or both of the first state and the second state in the electric signal described in (1) differs between the two electrodes, or
   set such that a duration of at least one of the first state, the second state, the third state, and the fourth state in the electric signal described in (2) differs between the two electrodes.
[Item 10'] The electrical stimulation method according to item 8', wherein
   an electric signal applied from one electrode pair of the two electrode pairs and an electric signal applied from the other electrode pair are
   set such that the application of a pulse wave in the first state is reversed between the two electrodes and the application of a pulse wave in the fourth state is reversed between the two electrodes in the electric signal described in (2).
[Item 11'] The electrical stimulation method according to any one of items 1' to 10', wherein a frequency of the electric signal is set within a range of 10 to 80 pps (pulses per second).
[Item 12'] The electrical stimulation method according to any one of items 1' to 11', wherein the electrical stimulation method is for treating brain dysfunction.

### Advantageous Effects of Invention

Using the electrical stimulator according to the present invention enables a more effective method of improving brain dysfunction to be implemented and, for example, even enables the IQ of a child with brain damage to be improved.

The electrical stimulator described in Patent Literature 2 is apparently capable of improving brain functions more effectively than the electrical stimulator that applies stimulation from peripheral nerves as described in Patent Literature 1 by stimulating the brain more effectively. However, the electrical stimulators described in these Patent Literatures are designed so that one electrode of a pair of electrodes is to be worn on the right half of the body and the other electrode of the pair of electrodes is to be on the left half of the body or, in other words, left and right electrodes form a pair at a hand or a foot in the case of Patent Literature 1 and in a region from the neck to the top of the head in the case of Patent Literature 2. When used in such a manner, electrical stimulation applied from one electrode (for example, the right half side) is sent from one hemisphere (for example, the right hemisphere) to the opposite hemisphere (for example, the left hemisphere) via the corpus callosum and flows to the other electrode (for example, the left half side). In the present invention, since information transmission through the corpus callosum differs from that in such prior arts and an amount of information passing through the corpus callosum can be increased, an advantageous effect that differs from that of the prior arts can be produced.

### Brief Description of Drawings

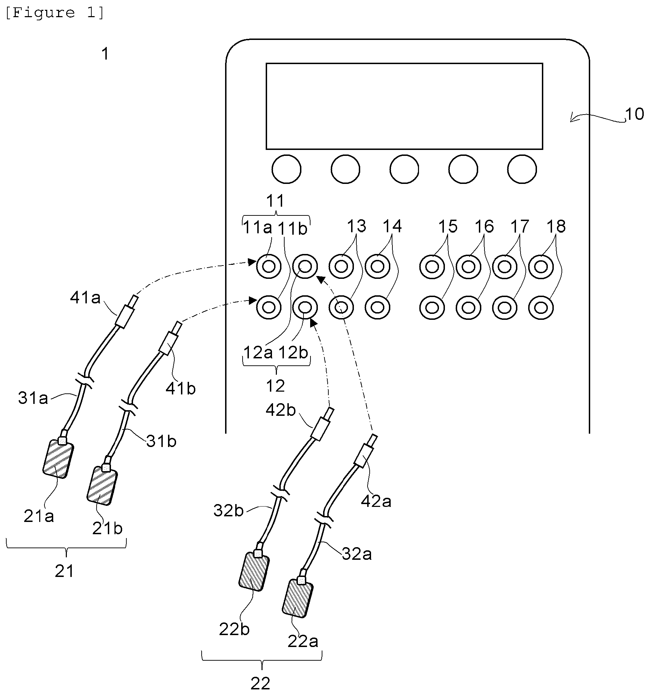

[Figure 1] Figure 1 is a simplified perspective view of an example of an electrical stimulator according to the present invention.

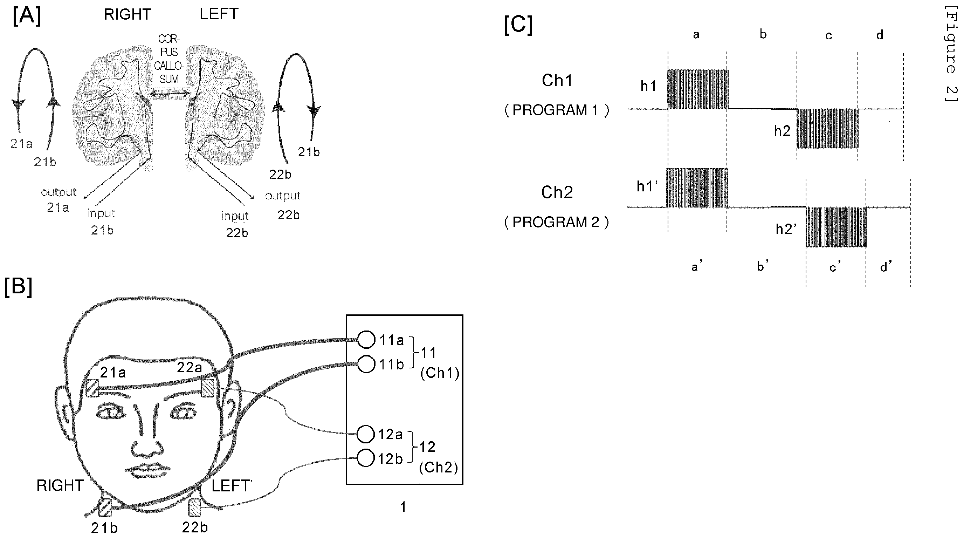

[Figure 2] Figure 2 is a schematic view of an embodiment of the present invention (a first embodiment of an electrical stimulation). In Figure **2****,** a to d represent lengths of first, second, third, and fourth states in an electrical stimulation applied by a first electrode pair that is connected to a first channel (Ch1), and a' to d' represent lengths of first, second, third, and fourth states in an electrical stimulation applied by a second electrode pair that is connected to a second channel (Ch2). In the present embodiment, since a = a' and c = c' but b # b' and d # d', there is a shift (asynchrony) in an electrical stimulation (pulse wave) applied. In addition, h1 and h2 represent current values of pulse waves in the first and second states in an electrical stimulation applied by the first electrode pair and h1' and h2' represent current values of pulse waves in the first and third states in an electrical stimulation applied by the second electrode pair. In the present embodiment, h1 = h2 and h1' = h2'.

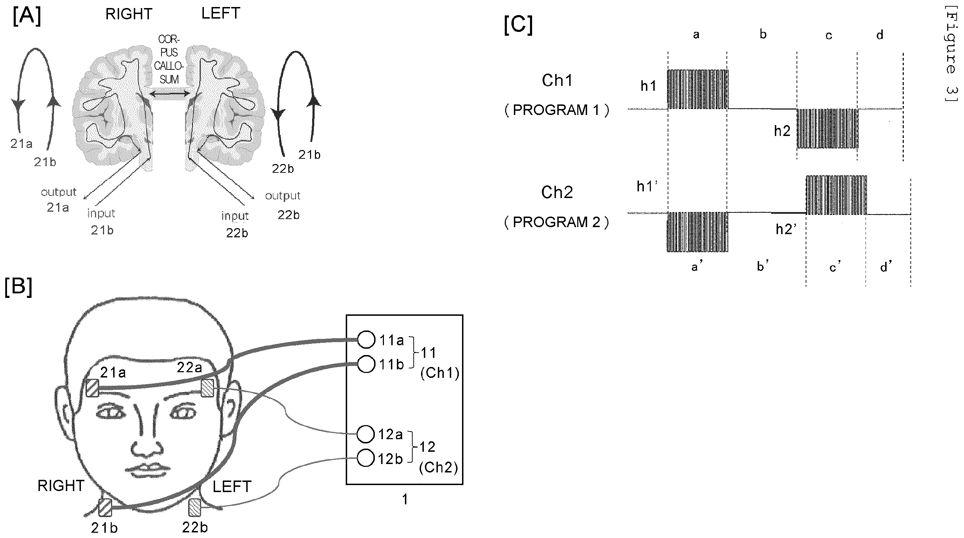

[Figure 3] Figure 3 is a schematic view of an embodiment of the present invention (a second embodiment of an electrical stimulation). In Figure 3, a to d, h1 and h2, a' to d', and h1' and h2' are similar to those in Figure 2. However, since the pulse wave in the first state in the electrical stimulation applied by the first electrode pair and the pulse wave in the first state in the electrical stimulation applied by the second electrode pair are in reverse in terms of positivity and negativity, and the pulse wave in the third state in the electrical stimulation applied by the first electrode pair and the pulse wave in the third state in the electrical stimulation applied by the second electrode pair are also in reverse in terms of positivity and negativity, the applied electrical stimulation (pulse waves) is not synchronized at all.

### Reference Signs List

- 1: electrical stimulator
- 10: main body
- 11: first channel (set of channels)
- 11a: first output unit of first channel
- 11b: second output unit of first channel
- 12: second channel (set of channels)
- 12a: first output unit of second channel
- 12b: second output unit of second channel
- 21: first electrode pair (one electrode pair)
- 21a: first electrode of first electrode pair
- 21b: second electrode of first electrode pair
- 31a: first cable of first electrode pair
- 31b: second cable of first electrode pair
- 41a: first terminal of first electrode pair
- 41b: second terminal of first electrode pair
- 22: second electrode pair (one electrode pair)
- 22a: first electrode of second electrode pair
- 22b: second electrode of second electrode pair
- 32a: first cable of second electrode pair
- 32b: second cable of second electrode pair
- 42a: first terminal of second electrode pair
- 42b: second terminal of second electrode pair

### Description of Embodiment

### - Electrical stimulator -

An electrical stimulator according to the present invention includes: a main body with at least two channels for outputting a set electric signal from a positive electrode output unit and a negative electrode output unit; at least two electrode pairs removably attached to a living body for applying the electric signal to the living body; and a cable for connecting at least two of channels of the main body and at least two electrodes to each other, wherein the two electrode pairs enable electrical stimulation to be applied to the brain while bypassing the brain stem by mounting one electrode pair to two specific positions on the right half of the body and mounting the other electrode pair to two specific positions on the left half of the body, the two electrode pairs are visually distinguishable from each other so that a difference in application of such mounting can be recognized, and the two electrode pairs apply mutually different electric signals to the specific positions.

Hereinafter, an embodiment of the present invention will be described in detail with reference to the drawings. Figure 1 shows a simplified perspective view of an example of the electrical stimulator according to the present invention.

An electrical stimulator 1 includes: a main body 10 that outputs a set electric signal; and two electrode pairs and cables, namely, a first electrode pair 21 (electrodes 21a and 21b) and a cable 31 (cables 31a and 31b, including terminal portions 41a and 41b, respectively) connecting the first electrode pair 21 and the main body 10 to each other, and a second electrode pair 22 (electrodes 22a and 22b) and a cable 32 (cables 32a and 32b, including terminal portions 42a and 42b, respectively) connecting the second electrode pair 22 and the main body 10 to each other. Note that an electric circuit for generating the set electric signal and which is connected to an output unit is provided inside the main body 10 (not illustrated).

The main body 10 includes a total of eight channels, namely, a first channel 11 (output units 11a and 11b), a second channel 12 (output units 12a and 12b), a third channel 13, ..., and an eighth channel 18, each channel (pair) constituted of two output units. While the main body 1 includes a total of eight channels in the embodiment shown in Figure 1, the main body 1 need only include at least two channels so that the first electrode pair 21 (electrodes 21a and 21b) and the second electrode pair 22 (electrodes 22a and 22b) can each connect to a channel. In each channel, one output unit is a positive electrode output unit and the other output unit is a negative electrode output unit. For example, in the first channel 11, the output unit 11a can be used as the positive electrode output unit and the output unit 11b can be used as the negative electrode output unit.

Two electrodes constitute a pair of electrodes. The electrodes 21a, 21b, 22a, and 22b become positive electrodes when connected to the positive electrode output unit of each channel of the main body 10 via their respective cables and terminal units and become negative electrodes when connected to the negative electrode output unit. A positively-applied electric signal (pulse wave) flows inside a living body from a positive electrode connected to the positive electrode output unit toward a negative electrode connected to the negative electrode output unit and, conversely, a negatively-applied electric signal flows inside a living body from the negative electrode connected to the negative electrode output unit toward the positive electrode connected to the positive electrode output unit. The electric signals of each channel can be independently controlled by a program.

In the present invention, as described separately, the electric signals (pulse waves) applied to the living body from one (first electrode pair) of the two electrode pairs and the other (second electrode pair) of the two electrode pairs have waveforms that differ from each other. However, the electric signals themselves applied to the living body from each electrode pair or, in other words, the electric signals themselves output from each channel of the main body 10 that is connected to each of the electrode pairs can be based on conventional electric signals.

The electric signal (pulse wave) applied to the living body can be set so as to repeat a cycle that includes a state in which the pulse wave is generated (referred to in the present specification as a "pulse wave generation state") and a state in which the pulse wave is inactive (referred to in the present specification as the "pulse wave inactive state").

In the pulse wave generation state, a frequency of the pulse wave is typically adjusted in the range of 10 to 80 pps (pulses per second) and can be set to 50 pps as an example. To allow the brain to perceive a single pulse, the frequency should normally be 80 pps or lower and preferably 60 pps or lower. On the other hand, since it becomes difficult to provide a sufficient number of stimulations when the frequency is too small, the frequency should normally be 10 pps or higher and preferably 20 pps or higher.

In addition, in the pulse wave generation state, a current value of the pulse wave may be constant, gradually increasing, or gradually decreasing. For example, a state in which the pulse wave is generated may consist of a state where the current value of the pulse wave gradually increases and a subsequent state in which the pulse wave is constant. The inclusion of a state in which the current value of the pulse wave gradually increases prevents a rapid electrical stimulation from being applied to the brain.

The current value of a pulse wave can be appropriately adjusted according to the embodiment of the present invention so that a suitable current flows inside the living body. For example, when the specific positions include two locations in the neck and head area (details will be provided later), the current value of the pulse wave is normally adjusted within the range of 1 to 1000 µA, and can be set to 40 µA as an example. To avoid over-stimulation of the brain, the current value is preferably 100 µA or less and more preferably 80 µA or less. Conversely, since the brain does not perceive current values that are too small, the current value is preferably 10 µA or more and more preferably 20 µA or more. In addition, when the specific positions include two locations outside of the neck and head area (hands, feet, back; details will be provided later), the current value of the pulse wave is normally adjusted within the range of 1 to 1000 mA, for example, the range of 10 to 500 mA, preferably the range of 30 to 300 mA.

Durations of the pulse wave generation state and the pulse wave inactive state, states in which the current value is constant, gradually increasing, and gradually decreasing in the pulse wave generation state, and the repetition of cycles can each be adjusted as necessary. For example, the pulse wave generation state and the pulse wave inactive state can each be adjusted within a range of 2 to 9 seconds. In addition, from the perspective of preventing brain fatigue, the duration of the pulse wave inactive state should be the same as or longer than the duration of the pulse wave generation state and, for example, the pulse wave inactive state can be made twice as long as the pulse wave generation state or longer.

In an embodiment of the present invention (a first embodiment of a pulse wave), the electric signal to be applied to the living body can be set so as to repeat a cycle including a first state in which positively-applied and negatively-applied pulse waves are alternately generated and a second state in which the pulse waves are inactive. The respective durations of the first state and the second state in the present embodiment can be adjusted, for example, within a range of 2 to 9 seconds.

In an embodiment of the present invention (a second embodiment of a pulse wave), the electric signal to be applied to the living body can be set so as to include, in this order, a first state in which a pulse wave that is always positively or negatively applied is generated, a second state in which the pulse wave is inactive, a third state in which a pulse wave applied in reverse to the first state is generated, and a fourth state in which the pulse wave is inactive. The current value of the positively-applied or negatively-applied pulse wave in the first state and the current value of the pulse wave applied in reverse in the third state may be set so as to match each other. The respective durations of the first to fourth states can be appropriately adjusted, for example, within a range of 2 to 9 seconds.

The first electrode pair 21 (electrodes 21a and 21b) and the second electrode pair 22 (electrodes 22a and 22b) are to be mounted to two specific positions on the right half of the body and two specific positions on the left half of the body, respectively, which enable electrical stimulation to be applied to the brain while bypassing the brain stem. Of the electrodes 21a and 21b in the first electrode pair 21 and the electrodes 22a and 22b in the second electrode pair 22, one is to be mounted to two specific positions on the right half of the body and the other is to be mounted to two specific positions on the left half of the body.

In the embodiment of the present invention, from the perspective of preventing stimulation from being reduced by the brain stem barrier by involving the peripheral nerves, the specific positions can include two locations in a region between the neck and the top of the head (referred to in the present specification as the "neck and head area"). For example, the specific region of one of the two electrode pairs can be two locations on the right half of the neck and head area (referred to in the present specification as the "right neck and head area") and the specific region of the other of the two electrode pairs can be two locations on the left half of the neck and head area (referred to herein as the "left neck and head area"). For example, the specific positions in each of the right neck and head area and the left neck and head area are preferably two locations selected from the group consisting of the temple, the forehead above the eye, the cheek, and the back of the neck. As an example, an embodiment may be adopted in which, of the first electrode pair, the electrode 21a is mounted to the temple of the right neck and head area and the electrode 21b is mounted to the back of the neck in the right neck and head area, and of the second electrode pair, the electrode 22a is mounted to the temple of the left neck and head area and the electrode 22b is mounted to the back of the neck in the left neck and head area.

In the embodiment of the present invention, the specific positions can include two locations other than the neck and head area such as the region below the neck including a region from the palm to the back of a hand (a fingertip, the ball of the thumb, a digit of the hand, or the like), a region from the sole to the top of a foot, and a region of the back along the spine. For example, the specific region of one of the two electrode pairs can be two locations on the right half of the body other than the neck and head area and the specific region of the other of the two electrode pairs can be two locations on the left half of the body other than the neck and head area. Alternatively, the specific region of one of the two electrode pairs can be two locations in the neck and head area (for example, on the right half of the body) and the specific region of the other of the two electrode pairs can be two locations other than the neck and head area (for example, on the left half of the body).

In the embodiment of the present invention, the specific positions may be a combination including at least two locations in the neck and head area such as two locations in the right neck and head area and two locations in the left neck and head area and, subsidiarily, further including at least two locations other than the neck and head area such as two locations on the right half of the body below the neck and two locations on the left half of the body below the neck. In an embodiment using such a combination, for example, the electrical stimulator can include the first electrode pair 21 and the second electrode pair 22 as electrode pairs for the right neck and head area and the left neck and head area, respectively and, subsidiarily, further include a third electrode pair (not illustrated) and a fourth electrode pair (not illustrated) as electrode pairs for the right half of the body and the left half of the body other than the neck and head area, respectively.

The first electrode pair 22 electrodes (21a and 21b) and the second electrode pair 22 (electrodes 22a and 22b) are made distinguishable from each other so that the application of one electrode pair for the right half of the body and the application of the other for the left half of the body can be recognized. By doing so, it becomes easier to avoid situations such as confusing the combination of the electrode 21a which constitutes the first electrode pair and the electrode 22b which constitutes the second electrode pair as a pair of electrodes. Furthermore, the first electrode pair 21a and 21b can both be more reliably mounted to specific positions on the right half of the body or the left half of the body and the second electrode pair 22a and 22b can both be more reliably mounted to specific positions on an opposite side. Note that the third electrode pair and the fourth electrode pair exemplified as auxiliary electrode pairs in the embodiment described above or other electrode pairs can similarly be made visually distinguishable from each other so that the application of one electrode pair for the right half of the body and the application of the other for the left half of the body can be recognized.

While a means for making "visually distinguishable from each other" is not particularly limited and various known means can be used, examples include means for making visually distinguishable mainly visually such as by color, labels with texts or symbols, and different shapes of electrodes. Note that cables (including terminal portions) and/or channels may also be made visually distinguishable together with the electrodes. For example, the first electrode pair 22 (electrodes 21a and 21b) and, when necessary, the cables 31a and 31b and the first channel 11 (output units 11a and 11b) to which the cables 31a and 31b connect may be colored red or marked with letters such as "right," while the second electrode pair 22 (electrodes 22a and 22b) and, when necessary, the cables 32a and 32b and the second channel 12 (output units 12a and 12b) to which the cables 32a and 32b connect may be colored blue or marked with letters such as "left". In addition, arranging electrodes on a fitting as described below also falls under means for making "visually distinguishable from each other".

The properties of each electrode (material, shape, and the like) are not particularly limited as long as the electrode can be removably attached to the living body and, for example, the electrode can be a pad-shaped electrode (gel electrode pad) made of a gel-like member. In addition, the electrodes to be mounted to the right neck and head area and the left neck and head area and the electrodes to be arranged on a fitting can be sheet-shaped while the electrodes to be worn on the right half of the body and the left half of the body other than the neck and head area (for example, an auxiliary electrode in the embodiment to be described later) and not to be arranged on the fitting can be suction cup-shaped.

In the present invention, the first electrode pair and the second electrode pair apply mutually different electric signals to the specific positions. Typically, mutually different electric signals can be applied to the specific positions by having the first channel 11 (output units 11a and 11b) and the second channel 12 (output units 12a and 12b) which are connected to the first electrode pair 21 (electrodes 21a and 21b) and the second electrode pair 22 (electrodes 22a and 22b) via cables output electric signals with waveforms that differ from each other. On the other hand, mutually different electric signals can be applied to the specific positions by making the specific positions for mounting the first electrode pairs 21a and 21b and the specific positions for mounting the second electrode pairs 22a and 22b mutually different from each other (for example, reversing the specific positions).

"Electric signals with waveforms that differ from each other" are not particularly limited and need only be electric signals that can increase the amount of information sent from each of the left and right hemispheres of the brain to the opposite hemisphere via the corpus callosum as compared to electric signals of a same waveform.

In the embodiment of the present invention (the first embodiment of a pulse wave), the electric signal (pulse wave) of the first electrode pair 21 and the electric signal (pulse wave) of the second electrode pair 22 are set so as to differ during at least one of the pulse wave generation state and the pulse wave inactive state.

For example, in the first embodiment of the electrical stimulation, the duration of either one of or both the first state (pulse wave generation state) and the second state (pulse wave inactive state) in the first embodiment of the pulse wave of the present invention described earlier can be made different, for example, the length of the first state can be made equal for both electrode pairs (for example, both set to three seconds) while the length of the second state can be made different from each other (for example, three seconds for the first electrode pair and five seconds for the second electrode pair). In addition, in the first embodiment of the electrical stimulation, the duration of at least one of the first state and the third state (pulse wave generation state) and the second state and the fourth state (pulse wave inactive state) in the second embodiment of the pulse wave of the present invention described earlier can be made different, for example, the lengths of the first state and the third state can be made equal for both electrode pairs while the lengths of the second state and the fourth state can be made different from each other (the lengths of the second state and the fourth state are made the same in each electrode pair).

As shown in Figure **2****,** in the first embodiment of electrical stimulation, adopting the settings described above causes the timing of pulse wave generation between the first electrode pair and the second electrode pair to shift (become asynchronous) or, in other words, electrical stimulation to the left and right hemispheres of the brain gradually shifts in timing even if a magnitude of the current value of the pulse wave is the same and a vector of the electrical stimulation is in the same direction. Since such information is sent by the left and right hemispheres of the brain to the opposite hemisphere, the need to exchange information via the corpus callosum increases, thereby stimulating the metabolism of nerve fibers in the corpus callosum, resulting in an enlargement of nerve fibers and formation of new nerve products which enable brain dysfunction to be improved more effectively. When the durations of the plurality of states of the pulse wave generation state and the pulse wave inactive state are set to differ in each cycle, while the duration of an entire cycle may be equal for each cycle, preferably, the duration of the entire cycle is also set to differ for each cycle. Even if the pulse waves are misaligned and become asynchronous between the first electrode pair and the second electrode pair, after a certain period of time, a moment will eventually arrive at which the pulse waves coincide and synchronize and, subsequently, a cycle of the pulse waves becoming asynchronous again and then returning to synchronization will be repeated.

In the other embodiment of the present invention (the second embodiment of electrical stimulation), the electric signal (pulse wave) of the first electrode pair 21 and the electric signal (pulse wave) of the second electrode pair are set such that the pulse waves in the pulse wave generation state are mutually applied in reverse.

For example, in the second embodiment of electrical stimulation, the application of the pulse wave in the first state and the third state in the second embodiment of the pulse wave of the present invention described above is set to be mutually in reverse between the two electrode pairs. As an example, the first state in the electric signal of the first electrode pair 21 can be set to be positively-applied, the first state in the electric signal of the second electrode pair 22 can be set to be negatively-applied, the third state in the electric signal of the first electrode pair 21 can be set to be negatively-applied, and the third state in the electric signal of the second electrode pair 22 can be set to be positively-applied. Note that the duration of each of the first state and the third state (pulse wave generation state) as well as the duration of each of the second state and the fourth state (pulse wave inactive state) may be matched between the two electrode pairs.

As shown in Figure 3, in the second embodiment of the electrical stimulation, adopting the settings described above result in a vector of the electrical stimulation being in an opposite direction and the electrical stimulation to the left and right hemispheres of the brain being independent. As an example, while a positive pulse wave is applied by the first electrode pair 21 and electrical stimulation flows in a direction from the electrode 21a (positive electrode) mounted to the right temple toward the electrode 21b (negative electrode) mounted to the back of the neck on the right side, a negative pulse wave is applied by the second electrode pair 22 and electrical stimulation flows in a direction from the electrode 22a (negative electrode) mounted to the back of the neck on the left side toward the electrode 22b (positive electrode) mounted to the left temple. Therefore, the left and right hemispheres of the brain send, as information to the opposite hemisphere, information which differs from the first embodiment of electric signals described above. In this case, since the need to exchange information via the corpus callosum also differs from the first embodiment, in addition to the enlargement of nerve fibers and the formation of new nerve products, new nerve products that cannot be formed in the first embodiment can now be formed and brain dysfunction can be improved more effectively.

In the embodiment of the present invention, the two electrode pairs may be arranged in advance at positions corresponding to the specific positions in a headgear-type fitting to be worn on a wearer's head. Since a wearer wearing the headgear-type fitting on his/her head can naturally cause one electrode pair (for example, the first electrode pair) to be arranged on the right neck and head area side and the other electrode pair (for example, the second electrode pair) to be arranged on the left neck and head area side, the present embodiment is suitable in an embodiment in which two electrode pairs are mounted to specific regions in the left and right neck and head areas (for example, two locations among the temple, the forehead above the eye, the cheek, and the back of the neck).

The headgear-type fitting is formed so as to be largely stretchable by, for example, an elastic sheet material. Specifically, the headgear-type fitting includes a bag-shaped main body part that can be worn over the wearer's head and neck and a front side of the main body part (patient face side) can be formed so that the wearer's eyes, nose, and mouth are exposed. The main body part is to cover the top of the head, left and right sides of the head, and the back of the head (including the upper portion of the neck) of the wearer. Two electrode pairs are arranged on an inner surface of the fitting in either a fixed manner or a removable manner (for example, using a hook and loop fastener) at positions corresponding to specific positions in the neck and head area. In addition, the main body part can be provided with a pair of left and right extensions that extend from a position slightly below the wearer's eyes toward the nose. Furthermore, by providing elastic strings that connect the pair of left and right extensions and elastic strings that connect the front lower ends of the main body part to each other, each electrode pair can be securely mounted to a specific position when the fitting is worn. A thin elastic plate can be held within the extensions, and the elasticity of this elastic plate can be used to ensure that the extensions adhere to the wearer's cheek area. Other matters related to the headgear-type fitting in the present invention can be appropriately designed by those skilled in the art by referring to known headgear-type fittings.

In the embodiment of the present invention, the two electrode pairs may be arranged in advance at positions corresponding to the specific positions in a semi-glove-like or semi-sock-like fitting to be worn on a wearer's hand or foot. Since a wearer wearing the fitting on his/her hand or foot can naturally cause one electrode pair (for example, the third electrode pair) to be arranged on the right half of the body other than the neck and head area and the other electrode pair (for example, the fourth electrode pair) to be arranged on the left half of the body other than the neck and head area, the present embodiment is suitable in an embodiment in which two electrode pairs are mounted to left and right specific regions other than the left and right neck and head areas.

For example, the semi-glove-like fitting has a main body part which can be fitted over the wearer's hand and which is made of, for example, a stretchy or elastic material. The semi-glove-like fitting may have fixing means such as a fastener or a hook and loop fastener for attaching to and detaching from the hand, in which case the semi-glove-like fitting need not necessarily be made of a stretchy material. Two electrode pairs are arranged at positions corresponding to regions at specific positions of the hand (the palm of a hand or the back of the hand) on an inner surface of the semi-glove-like fitting. For example, one electrode pair is each arranged at positions corresponding to the ball of the thumb (first specific region) and a fingertip (second specific region) on the inner surface of the semi-glove-like fitting (main body part) for each of the right hand and the left hand. Other matters related to the semi-glove-like or semi-sock-like fitting in the present invention can be appropriately designed by those skilled in the art by referring to known semi-glove-like or semi-sock-like fittings.

### <Brain dysfunction>

The electrical stimulator according to the present invention can be used to treat brain dysfunction. In other words, in an embodiment of the present invention, the electrical stimulator according to the present invention is a medical device for treating brain dysfunction. The term "treatment" as used in the present specification includes completely or partially treating brain dysfunction, completely or partially curing (alleviating or ameliorating) symptoms associated with brain dysfunction, slowing the progression, delaying the occurrence, preventing the occurrence, or reducing the risk of occurrence, of brain dysfunction or its symptoms, or a combination of these definitions. Treatments may involve nonclinical, preclinical, or clinical trials.

Brain dysfunctions that can be treated using the electrical stimulator according to the present invention or, in other words, brain dysfunctions on which the electrical stimulator according to the present invention can produce an ameliorative effect include, for example, the following:
as organic mental disorders including symptomatic mental disorders, mental illnesses due to large (i.e., visible to the eye) lesions of the brain such as dementia, Korsakoff's syndrome, and sequelae of head trauma;
as mental and behavioral disorders due to psychoactive substance use, psychiatric disorders (divided into dependence, abuse, addiction, and the like) related to psychoactive substances such as alcohol, opiates, cannabis, sedatives or hypnotics, cocaine, stimulants/caffeine, hallucinogens, tobacco, volatile solvents and sugar, alcoholism, drug addiction, and the like;
as schizophrenia, schizotypal disorder, and delusional disorder, schizophrenia, schizotypal disorder, persistent delusional disorder, acute transient psychotic disorder, sensorimotor delusional disorder, and schizoaffective disorder;
as mood disorders (also called affective disorders, which primarily affect mood), mania, bipolar disorder (manic-depressive illness), and depression;
as neurotic disorders, stress-related disorders, and somatoform disorders, phobic anxiety disorders (agoraphobia [syndrome], social phobia [syndrome]), other anxiety disorders (panic disorder, generalized anxiety disorder), obsessive-compulsive disorder, severe stress disorder, severe stress response and application disorder (PTSD and acute stress disorder are included in anxiety disorders in DSM-IV but classified as severe stress reactions in ICD-10), acute stress disorder, post-traumatic stress disorder (PTSD), adjustment disorder, dissociative (conversion) disorder (F44), dissociative disorder (approximately corresponds to dissociative disorder in DSM IV-TR), other dissociative (conversion) disorders, multiple personality disorder (dissociative identity disorder in DSM-IV-TR), somatoform disorders (somatization disorder, psychosomatic disorder, persistent somatoform pain disorder), and other neurotic disorders;
as behavioral syndromes associated with physiological disorders and physical factors, eating disorders, anorexia nervosa (anorexia), bulimia nervosa (bulimia), sleep disorders, insomnia, psychophysiological insomnia (circadian rhythm sleep disorder, difficulty falling asleep, mid-wake, early morning awakenings, hypersomnia, sleep apnea syndrome, narcolepsy, primary hypersomnia, repetitive hypersomnia, and idiopathic hypersomnia), sleep paralysis, REM sleep behavior disorder, sleepwalking, and night terrors;
as personality and behavior disorders, delusional personality disorder, schizoid personality disorder, asocial personality disorder, emotionally unstable personality disorder (impulsive and borderline), acting personality disorder, obsessive-compulsive personality disorder, anxious [avoidant] personality disorder, dependent personality disorder, other (or unspecified or mixed) personality disorders, persistent personality changes, personality and behavior disorders not due to brain injury and brain disease, habit and impulse disorders, gender identity disorders, sexual preference disorders (fetishism, exhibitionism, voyeurism, pedophilia, sadism and masochism), psychological and behavioral disorders related to sexual development and orientation, other adult personality and behavioral disorders, intentional calculation or feigning of symptoms or incapacity, physical or psychological personality and behavioral disorders (falsity disorders), and Munchausen Syndrome;
as mental retardation, intellectual disability;
as disorders of psychological development, pervasive developmental disorders, autism, and Asperger's syndrome;
as behavioral and emotional disorders that usually develop during childhood and adolescence, hyperactivity disorder (ADHD) (tic disorder, Tourette's disorder); and
as other disorders, hallucinations, delusions, and cultural dependency syndrome (nervousness, anthropophobia).

### - Electrical stimulation method -

An electrical stimulation method according to the present invention is an electrical stimulation method that uses at least two electrode pairs, wherein the two electrode pairs enable electrical stimulation to be applied to the brain while bypassing the brain stem by mounting one electrode pair to two specific positions on the right half of the body and mounting the other electrode pair to two specific positions on the left half of the body, and the two electrode pairs apply mutually different electric signals to the specific positions. The two electrode pairs may be included in an electrical stimulator that includes: a main body with at least two channels for outputting a set electric signal from a positive electrode output unit and a negative electrode output unit; a cable for connecting the at least two channels of the main body and the at least two electrode pairs to each other; and at least two electrode pairs removably attached to a living body for applying the electric signal to the living body. In addition, the two electrode pairs may be visually distinguishable from each other so that a difference in application of mounting can be recognized. The electrical stimulation method according to the present invention can be implemented to treat (improve) brain dysfunction.

In the present specification, the technical matters related to the electrical stimulator described earlier can be applied as technical matters related to the electrical stimulation method. For example, a preferred embodiment related to the electrical stimulator can be converted into a preferred embodiment related to the electrical stimulation method. Conversely, in the present specification, the technical matters related to the electrical stimulation method can be applied as the technical matters related to the electrical stimulator.

### Examples

### [Example 1]

| | | |
|---|---|---|
| UH | Male | 5 years 7 months old |

Patient diagnosed with autism. From December 12, 2014 to August 31, 2015, the patient was treated with the electrical stimulator according to the present invention. The electrical stimulator was a headgear-type fitting equipped with electrodes that apply electrical stimulation to the temples and the back of the neck on each of left and right sides (the same applies to Example 2 below). Treatment frequency was 1 to 3 days/week. IQ was 58 before treatment and increased to 67 after treatment.

### [Example 2]

| | | |
|---|---|---|
| YF | Male | 11 years 9 months old |

Patient diagnosed with unspecified pervasive developmental disorder. From May 26, 2013 to June 10, 2016, the patient was treated with the electrical stimulator according to the present invention. Treatment frequency was 1 to 5 days/week. IQ was 74 before treatment and increased to 92 after treatment.

## Claims

1. An electrical stimulator, comprising:
a main body with at least two channels for outputting a set electric signal from a positive electrode output unit and a negative electrode output unit;
at least two electrode pairs removably attached to a living body for applying the electric signal to the living body; and
a cable for connecting the at least two channels of the main body and the at least two electrode pairs to each other, wherein
the two electrode pairs enable electrical stimulation to be applied to the brain while bypassing the brain stem by mounting one electrode pair to two specific positions on the right half of the body and mounting the other electrode pair to two specific positions on the left half of the body, the two electrode pairs are visually distinguishable from each other so that a difference in application of such mounting can be recognized, and the two electrode pairs apply mutually different electric signals to the specific positions.

2. The electrical stimulator according to claim 1, wherein the specific positions include two locations in the neck and head area selected from the group consisting of the temple, the forehead above the eye, the cheek, and the back of the neck.

3. The electrical stimulator according to claim 2, wherein the two electrode pairs are arranged in advance at positions corresponding to the specific positions including two locations in the neck and head area in a headgear-type fitting to be worn on a wearer's head.

4. The electrical stimulator according to claim 2, wherein a current value of the electric signal applied to the specific positions including two locations in the neck and head area is set within a range of 1 to 1000 µA.

5. The electrical stimulator according to claim 1, wherein the specific positions include two locations other than the neck and head area selected from the group consisting of a region from the palm to the back of a hand, a region from the sole to the top of a foot, and a region of the back along the spine.

6. The electrical stimulator according to claim 5, wherein the two electrode pairs are arranged in advance at positions corresponding to the specific positions including two locations other than the neck and head area in a semi-glove-like or semi-sock-like fitting to be worn on a wearer's hand or foot.

7. The electrical stimulator according to claim 5, wherein a current value of the electric signal applied to the specific positions including two locations other than the neck and head area is set within a range of 1 to 1000 µA.

8. The electrical stimulator according to claim 1, wherein
the electric signal applied to the specific positions is:
(1) set so as to repeat a cycle including a first state in which positively-applied and negatively-applied pulse waves are alternately generated and a second state in which the pulse waves are inactive; or
(2) set so as to include, in this order, a first state in which a pulse wave that is always positively or negatively applied is generated, a second state in which the pulse wave is inactive, a third state in which a pulse wave applied in reverse to the first state is generated, and a fourth state in which the pulse wave is inactive.

9. The electrical stimulator according to claim 8, wherein
an electric signal applied from one electrode pair of the two electrode pairs and an electric signal applied from the other electrode pair are
set such that a duration of either one or both of the first state and the second state in the electric signal described in (1) differs between the two electrodes, or
set such that a duration of at least one of the first state, the second state, the third state, and the fourth state in the electric signal described in (2) differs between the two electrodes.

10. The electrical stimulator according to claim 8, wherein
an electric signal applied from one electrode pair of the two electrode pairs and an electric signal applied from the other electrode pair are
set such that the application of a pulse wave in the first state is reversed between the two electrodes and the application of a pulse wave in the fourth state is reversed between the two electrodes in the electric signal described in (2).

11. The electrical stimulator according to claim 1, wherein a frequency of the electric signal is set within a range of 10 to 80 pps (pulses per second).

12. The electrical stimulator according to claim 1, wherein the electrical stimulator is a medical device for treating brain dysfunction.
